# EUROPEAN PATENT APPLICATION

(11) **EP 2 044 887 A1**
(43) Date of publication of application: **08.04.2009**
(21) Application number: 08017337.0
(22) Date of filing: 02.10.2008
(51) Int. Cl.: A61B 8/00, G01S 7/521, G06F 1/16

(54) **Portable ultrasonic diagnostic device**

(30) Priority: 02.10.2007 KR 20070099449; 22.09.2008 KR 20080092884
(71) Applicant: MEDISON CO., LTD., Kangwon-do 250-870 (KR)
(72) Inventor: Shin, Soo Hwan, Seoul 135-280 (KR); Song, Young Seuk, Seoul 135-280 (KR); Lee, Sun Ki, Seoul 135-280 (KR)
(74) Representative: Schmid, Wolfgang

(57) **Abstract**

There is provided a portable ultrasonic diagnostic device (100) wherein a handle (130) for easy carriage or transportation is removably coupled to a main body (110). The portable ultrasonic diagnostic device (100) comprises: a main body (110) having two fixing holes (111) at one edge thereof; a handle (130), one end portion and the opposite end portion thereof being adapted to be inserted into the main body (110) through the two fixing holes (111); and a device for removably coupling the one end portion and the opposite end portion of the handle (130) to the main body (110).

## Description

The present application claims priority from Korean Patent Application No. 10-2007-99449 filed on October 2, 2007 and Korean Patent Application No. 10-2008-92884 filed on September 22, 2008, the entire subject matters of which are incorporated herein by reference.

### BACKGROUND

### 1. Field

Embodiments of the present invention may relate to a portable ultrasonic diagnostic device.

### 2. Background

An ultrasonic diagnostic device is a medical imaging equipment for diagnosing a subject by processing echo signals obtained by radiating ultrasonic waves through an ultrasonic probe and visualizing a portion within the subject's body. The ultrasonic diagnostic device generally has an ultrasonic probe, which comprises a transducer for converting electric signals into ultrasonic waves or *vice versa*. Such an ultrasonic diagnostic device is advantageously used for obtaining clinical information of the subject (e.g., lesion or neoplasm information of internal organs, fetus information, etc.).

With the miniaturization of the ultrasonic diagnostic device, a portable ultrasonic diagnostic device of a notebook computer and the like has been developed. However, such portable ultrasonic diagnostic device has no handle, thereby failing to be easily carried or transported.

Further, gel is also used in ultrasonic diagnosis in addition to probes. However, the portable ultrasonic diagnostic device of the prior art is not configured to receive the probe or a gel vessel containing the gel. This makes it inconvenient to use the gel or the probe. Particularly, since a scan head of the probe is very sensitive, the probe must be carefully kept when it is not in use.

### BRIEF DESCRIPTION OF THE DRAWINGS

Arrangements and embodiments may be described in detail with reference to the following drawings in which like reference numerals refer to like elements and wherein:

Fig. 1 is a perspective view showing an embodiment of a portable ultrasonic diagnostic device in accordance with the present invention;

Fig. 2 is a perspective view showing the open portable ultrasonic diagnostic device.

Fig. 3 is a perspective view showing another embodiment of a portable ultrasonic diagnostic device in accordance with the present invention;

Fig. 4 is a partial perspective view showing an engagement between a main body and a handle.

Fig. 5 is a perspective view showing an engagement between a handle and a holder;

Fig. 6 is a perspective view of a handle having protrusions for preventing slippage; and

Fig. 7 is a perspective view of a handle with a convex grip portion thereon.

### DETAILED DESCRIPTION OF THE PRESENT INVENTION

A detailed description may be provided with reference to the accompanying drawings. One of ordinary skill in the art will appreciate that the following description is illustrative only and is not in any way limiting. Other embodiments of the present invention may readily suggest themselves to such skilled persons having the benefit of this disclosure.

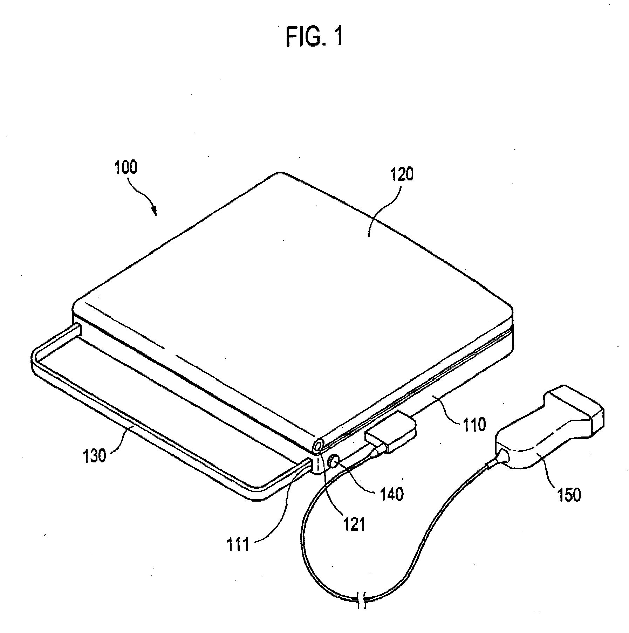

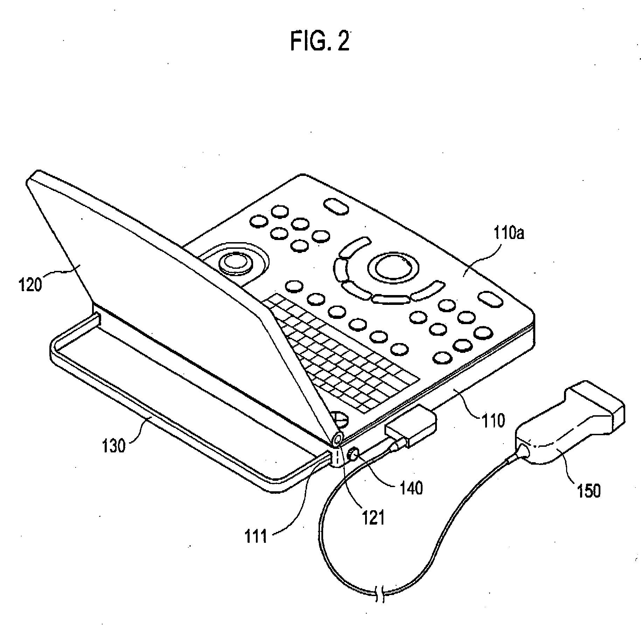

Fig. 1 is a perspective view showing an embodiment of a portable ultrasonic diagnostic device in accordance with the present invention. As shown in Fig. 1, the portable ultrasonic diagnostic device 100 of this embodiment may take the shape of a notebook computer. The portable ultrasonic diagnostic device 100 comprises: a plate-shaped main body 110; a display device 120 hinge-jointed to one side edge of the main body 110 for displaying ultrasound images; and a probe 150 connected to another side edge of the main body 110 for carrying out ultrasonic probing. The main body 110 may contain electronic components for carrying out the ultrasonic diagnosis. On an upper face 110a of the main body 110, which faces the display device 120, there is provided input devices for inputting commands needed for the ultrasonic diagnosis. The display device 120 may include a flat panel display device such as an LCD. The display device 120 may be joined to the one side edge of the main body 110 via a hinge device 121 to thereby be opened and closed relative to the main body 110 (*see* Fig. 2).

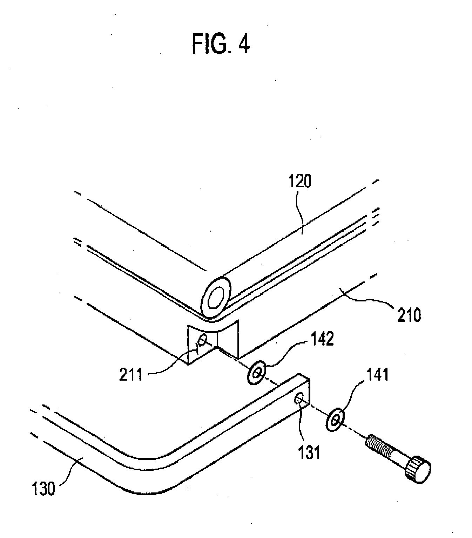

The portable ultrasonic diagnostic device 100 further comprises a handle 130 for easy carriage and transportation. The handle 130 may be removably coupled to the main body 110. The handle 130 may be generally U-shaped and may be fabricated from a plastic material or any hard material. In order to couple the handle 130 to the main body 110, the main body 110 may have two fixing holes, through which one end portion and the opposite end portion of the handle 130 are inserted into the main body, at its one side edge. More specifically, the main body 110 includes a case forming its external appearance. One side edge of the case, along which the hinge device 121 is disposed, is formed with the two fixing holes 111 at a predetermined interval. Each of the one and the opposite end portions of the handle 130 is formed with through-holes 131 (shown in Figs. 4 to 7). The one and the opposite end portions of the handle 130 are inserted through the fixing holes 111, while bolts 140 are fastened to the main body 110 through the through-holes 131, thereby coupling the handle 130 to the main body 110. To this end, the inside of the case of the main body 110 may be provided with bolt holes (not shown) that allow engagement of the bolt thereto. The bolts 140 can be loosened or fastened by a user. Thus, the handle 130 can be removably coupled to the main body 110.

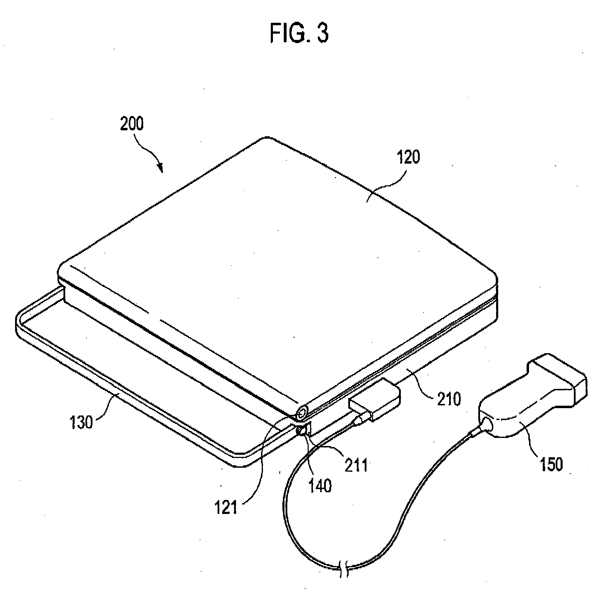

Fig. 3 is a perspective view showing another embodiment of a portable ultrasonic diagnostic device in accordance with the present invention. The portable ultrasonic diagnostic device 200 of this embodiment has the same configuration as the portable ultrasonic diagnostic device of the above-described embodiment except that a structure for coupling the handle 130 to the main body 110 is modified. A main body 210 of the portable ultrasonic diagnostic device 200 has two fixing recesses 211, on which the one and the opposite end portions of the handle 130 are placed, at its one side edge. More specifically, the main body 210 includes a case forming its external appearance. The two fixing recesses 211 are formed at corners between one side edge of the case, along which the hinge device 121 is disposed, and lateral side edges joining thereto. The one and the opposite end portions of the handle 130 are contacted to the fixing recesses 211 and the bolts 140 are fastened to the main body 110 through the through-holes 131 (e.g., engagement to bolt holes formed on the fixing recesses), thereby coupling the handle 130 to the main body 210. Since the bolts 140 can be loosened or fastened by a user, the handle 130 can be removably coupled to the main body 210.

In the above-described embodiments, the fixing holes 111 and the fixing recesses 211 are sized such that the one and the opposite end portions of the handle 130 can be pivoted therein. Thus, an angle between the handle 130 and the main body can be varied. The handle 130 may be coupled to the main body 110, 210 in a slanted and downward relationship therewith. Heat radiation caused by convection can be increased beneath the main body 110, 210. This is because the main body 110, 210 can be supported on the surface with any angle therebetween. By contrast, when the handle 130 is coupled to the main body 110, 210 as slanted upward relative the main body 110, 210, holders of a probe or a gel vessel, which will be described later, can be effectively attached to the handle 130.

Further, as shown in Fig. 4, a plurality of friction members 141 and 142 may be interposed between a head of the bolts 140 and either the one end portion or the opposite end portion of the handle 130 and between either the one end portion or the opposite end portion of the handle 130 and the main body 110, 210. In such a case, when the handle 130 may be pivoted or slanted relative to the main body, the handle 130 can be maintained due to the friction force caused by the friction members in such position. Said friction members 141 and 142 may include plain washers or spring washers.

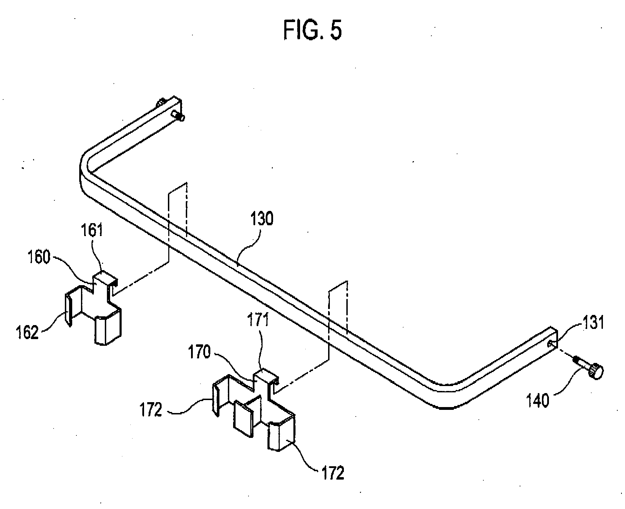

In one embodiment, the portable ultrasonic diagnostic device may further comprise a probe holder 170 for attaching the probe 150 to the handle 130 and a gel vessel holder 160 for attaching a gel vessel to the handle 130. Fig. 5 shows an engagement between the handle and the holders. Since the handle 130 is fabricated from a plastic material, it is possible to attach the gel vessel holder 160 or the probe holder 170 to the handle 130. The gel vessel holder 160 includes a hook-shaped engagement portion 161 adapted to be caught or fitted to the handle 130 and a holding portion 162 for receiving the gel vessel therein. The probe holder 170 includes a hook-shaped engagement portion 171 adapted to be caught or fitted to the handle 130 and a holding portion 172 for receiving the probe 150 therein. In this embodiment, the holding portion 172 of the probe holder 170 may be configured so as to be suitable for receiving a scan head and a switch box of a probe. While the gel vessel holder 160 and the probe holder 170 may be used to attach the gel vessel and the probe to the handle 130 in this embodiment, a gel vessel or a probe may be modified so as to have any engagement portion to the handle 130 and such a gel vessel or a probe may be directly attached to the handle 130.

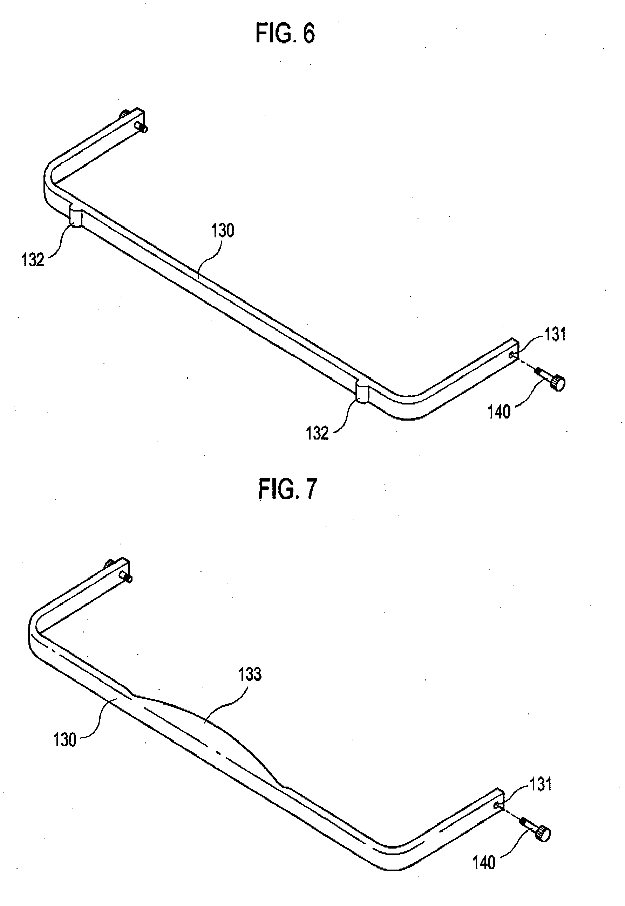

Fig. 6 shows the handle with protrusions for preventing slippage. Fig. 7 shows the handle with a convex grip portion thereon. The handle 130 may be used for carrying the portable ultrasonic diagnostic device. Since the portable ultrasonic diagnostic device is expensive and sensitive, it must be given more care when being carried or transported. As shown in Fig. 5, a body of the handle 130 may be formed with the protrusions 132 for preventing the handle 130 from slipping out of a user's hand. Further, as shown in Fig. 6, the convex grip portion 133 may be formed on a center of the handle 130, thereby mitigating the fatigue of a user's hand when a user grips the handle 130. Furthermore, even if not shown, the handle 130 may be formed with both the grip portion 133 and the protrusions 132.

The operational effects of the portable ultrasonic diagnostic device will now be described.

The handle 130 may be removably coupled to the main body 110, 210 by the bolts 140. In case of a long-distance transportation, the portable ultrasonic diagnostic device may be transported as accommodated in its specific bag. Further, in case of a short-distance transportation such as transportation in a hospital, it may be transported as being carried by means of the handle 130. Thus, when the handle 130 is used no longer, the handle 130 may be allowed to be removed from the main body 110, 210 and kept separately. Since the convex grip portion 133 is formed on the center of the handle 130 and the protrusions 132 for preventing slippage are formed beside the grip portion 133, the convenience of carriage can be improved.

Moreover, the gel vessel holder 160 and the probe holder 170 can be easily attached to the handle 130 via their engagement portions 162, 172. Thus, the probe or the gel vessel can be received by the holders attached to the handle 130, thereby providing convenience in use and maintaining the probe or gel vessel more safely. Especially, since a scan head of the probe is extremely sensitive, the probe can be safely maintained in the probe holder 170 attached to the handle 130.

In the embodiments described with reference to Figs. 1 to 4, since the friction members such as washers are provided among the bolts 140, the handle 130 and the main body 110, 210, the handle 130 can be maintained as pivoted relative to the main body 110, 210. The handle 130 can be pivoted downward relative to the main body 110, 210. In such a case, any angle is formed beneath the main body 110, 210, thereby increasing heat radiation caused by convection below the main body 110. Further, the handle 130 can be pivoted upward relative to the main body 110, 210. In such a case, the probe 150 can be easily retained to the handle 130 by attaching the probe holder 170 to the handle 130. Additional components other than the probe can also be attached to the handle 130.

A user can easily carry the portable ultrasonic diagnostic device by means of the handle removably coupled to the main body. Further, since the holders for supporting the gel vessel or the probe can be attached to the handle, the gel vessel or the probe can be easily kept in the portable ultrasonic diagnostic device lacking a storage space.

Embodiments of the present invention may provide a portable ultrasonic diagnostic device. The portable ultrasonic diagnostic device may comprise: a main body for carrying out ultrasonic diagnosis, the main body having two fixing holes at one side edge thereof; a handle, one end portion and the opposite end portion thereof being adapted to be inserted into the main body through the two fixing holes; and a device for removably coupling the one end portion and the opposite end portion of the handle to the main body. Further, the portable ultrasonic diagnostic device may comprise: a main body for carrying out ultrasonic diagnosis, the main body having two fixing recesses at one side edge thereof; a handle, one end portion and the opposite end portion thereof being adapted to contact the two fixing recesses; and a device for removably coupling the one end portion and the opposite end portion of the handle to the main body.

The removably coupling device may comprise a bolt fastenable to the main body through the one and the opposite end portions of the handle. The removably coupling device may further include washers interposed between the one and the opposite end portions of the handle and the main body and between the one and the opposite end portions of the handle and a head of the bolt.

The portable ultrasonic diagnostic device may further comprise at least one holder having an engagement portion engageable to the handle. The engagement portion of the holder may have a hook-shape.

The handle may have a convex grip portion at a central portion thereof. The handle may have a plurality of protrusions for preventing slippage.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other ones of the embodiments.

Although embodiments have been described with reference to a number of illustrative embodiments thereof, it should be understood that various other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A portable ultrasonic diagnostic device, comprising:
a main body 110 configured to accommodate electronic elements for ultrasonic processing, the main body 110 having two fixing holes 111 at one side edge thereof;
a handle 130, one end portion and an opposite end portion thereof being adapted to be inserted into the main body 110 through the two fixing holes 111; and
a device configured to removably couple the one end portion and the opposite end portion of the handle 130 to the main body 110.

2. A portable ultrasonic diagnostic device, comprising:
a main body 210 configured to accommodate electronic elements for ultrasonic processing, the main body 210 having two fixing recesses 211 at one side edge thereof;
a handle 130, one end portion and an opposite end portion thereof being adapted to contact the two fixing recesses 211; and
a device configured to removably couple the one end portion and the opposite end portion of the handle 130 to the main body 210.

3. The portable ultrasonic diagnostic device of Claim 1 or 2, wherein the removably coupling device comprises a bolt 140 fastenable to the main body 110, 210 through the one and opposite end portions of the handle 130.

4. The portable ultrasonic diagnostic device of Claim 3, wherein the removably coupling device further includes washers interposed between the one and opposite end portions of the handle 130 and the main body 110, 210 and between the one and the opposite end portions of the handle 130 and a head of the bolt 140.

5. The portable ultrasonic diagnostic device of Claim 1 or 2, further comprising at least one holder 160, 170 having an engagement portion engageable to the handle 130.

6. The portable ultrasonic diagnostic device of Claim 5, wherein the engagement portion of the holder 160, 170 has a hook-shape.

7. The portable ultrasonic diagnostic device of Claim 1 or 2, wherein the handle 130 has a convex grip portion 133 at a central portion thereof.

8. The portable ultrasonic diagnostic device of Claim 1 or 2, wherein the handle 130 has a plurality of protrusions 132 for preventing slippage.
